(19) **Europäisches Patentamt**
**European Patent Office**
**Office européen des brevets**

(11) **EP 4 517 776 A1**

(12) **EUROPEAN PATENT APPLICATION**

(43) Date of publication:
**05.03.2025 Bulletin 2025/10**

(21) Application number: **24197236.3**

(22) Date of filing: **29.08.2024**

(51) International Patent Classification (IPC):
**G16H 50/20** (2018.01)    **G16H 50/30** (2018.01)

(52) Cooperative Patent Classification (CPC):
**G16H 50/20; G16H 50/30;** G06N 3/045;
G16H 50/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 US 202318459604**

(71) Applicant: **Siemens Healthcare Diagnostics Inc.**
**Tarrytown, NY 10591 (US)**

(72) Inventors:
• **Singh, Vivek**
**Princeton, NJ, 08540 (US)**
• **Kamen, Ali**
**Skillman, NJ, 08558 (US)**
• **Gopalan, Raj**
**Durham, NC, 27705 (US)**
• **Gramz, Jamie**
**Cranberry Township, PA, 16066 (US)**
• **Comaniciu, Dorin**
**Princeton, NJ, 08540 (US)**

(74) Representative: **Schweitzer, Klaus**
**Plate Schweitzer Zounek**
**Patentanwälte**
**Rheingaustrasse 196**
**65203 Wiesbaden (DE)**

(54) **CLINICAL DECISION SUPPORT USING TRANSFORMER-BASED NETWORKS BY IMPUTING BIOMARKERS**

(57)    Systems and methods for performing one or more medical analysis tasks are provided. Patient data of a patient is received for a set of biomarkers acquired at one or more time points within a period of time. The patient data is encoded using an encoder network to generate patient data embeddings. One or more medical analysis tasks are performed based on the patient data embeddings using one or more decoder networks. The one or more medical analysis tasks comprise generating recommendations for a clinical course of action. Results of the one or more medical analysis tasks are output.

EP 4 517 776 A1

FIG 1

**Description**

TECHNICAL FIELD

The present invention relates generally to clinical decision support, and in particular to clinical decision support using transformer-based networks by imputing biomarkers.

BACKGROUND

With the high prevalence and high mortality associated with cancer, significant research has been conducted to help identify patients at higher risk of cancer. In the current clinical workflow, patients that are at a higher risk of cancer are identified based on, for example, medical conditions (e.g., diabetes), genetic markers, and family medical history. Recently, various screening procedures have been developed to facilitate early diagnosis of cancer, such as, e.g., FIT (fecal immunochemical test) and colonoscopy for colorectal cancer and mammography for breast cancer. While these advances have been instrumental in diagnosing patients and developing the treatment pathway, there is still a need for increased awareness and early identification of cancer and other rapidly progressing diseases so that treatment or intervention can start earlier.

BRIEF SUMMARY OF THE INVENTION

In accordance with one or more embodiments, systems and methods for performing one or more medical analysis tasks are provided. Patient data of a patient is received for a set of biomarkers acquired at one or more time points within a period of time. The patient data is encoded using an encoder network to generate patient data embeddings. One or more medical analysis tasks are performed based on the patient data embeddings using one or more decoder networks. The one or more medical analysis tasks comprise generating recommendations for a clinical course of action. Results of the one or more medical analysis tasks are output.

In one embodiment, the patient data is missing data for certain biomarkers of the set of biomarkers for certain time points within the period of time. The one or more medical analysis tasks are performed by imputing the missing data for the certain biomarkers for the certain time points.

In one embodiment, the patient data is encoded with time embeddings defining a relative temporal position relative to a given reference time. The patient data is encoded with the time embeddings using the encoder network to generate the patient data embeddings.

In one embodiment, the encoder network comprises a transformer-based encoder network and the one or more decoder networks comprise one or more transformer-based decoder networks.

In one embodiment, the one or more medical analysis tasks comprise determining a risk score associated with a disease. In another embodiment, the one or more medical analysis tasks comprise determining a confidence interval associated with another task of the one or more medical analysis tasks.

In one embodiment, a user interface depicting a likelihood ratio score and a cohort of patients similar to the patient is presented.

In one embodiment, the encoder network and the one or more decoder networks are trained by simulating certain data for one or more biomarkers of the set of biomarkers as being missing in the training patient data. In another embodiment, the encoder network and the one or more decoder networks are trained using deep reinforcement learning by iterating over a window of training patient data and estimated future patient data. The estimated future patient data is estimated by a machine learning based model, the machine learning based model receiving as input the training patient data and generating as output the estimated future patient data.

These and other advantages of the invention will be apparent to those of ordinary skill in the art by reference to the following detailed description and the accompanying drawings.

BRIEF DESCRIPTION OF THE DRAWINGS

Figure 1 shows a schematic representation of the health assessment of a patient within a period of time, in accordance with one or more embodiments;

Figure 2 shows a method for performing one or more medical analysis tasks for clinical decision support, in accordance with one or more embodiments;

Figure 3 shows an architectural diagram of an AI (artificial intelligence) based system for performing one or more medical analysis tasks for clinical decision support, in accordance with one or more embodiments;

Figure 4 shows a user interface presenting results of the one or more medical analysis tasks, in accordance with one or more embodiments;

Figure 5 shows a workflow for training a decoder network for imputing missing data, in accordance with one or more embodiments;

Figure 6 shows a workflow for training an encoder network and one or more decoder networks for performing one or more medical analysis tasks for clinical decision support using deep reinforcement learning, in accordance with one or more embodiments;

Figure 7 shows a network architecture for a state transition model for estimating future patient data, in accordance with one or more embodiments;

Figure 8 shows an exemplary artificial neural network that may be used to implement one or more embodiments;

Figure 9 shows a convolutional neural network that may be used to implement one or more embodiments; and

Figure 10 shows a high-level block diagram of a computer that may be used to implement one or more embodiments.

DETAILED DESCRIPTION

The present invention generally relates to methods and systems for clinical decision support using transformer-based networks by imputing biomarkers. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

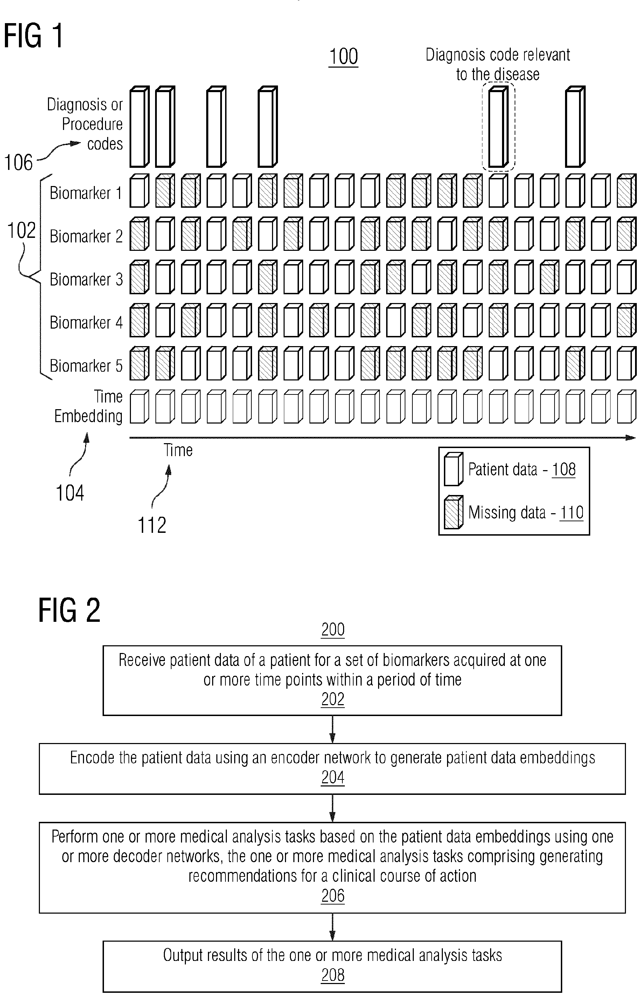

Figure 1 shows a schematic representation of the health assessment 100 of a patient over a period of time 112, in accordance with one or more embodiments. As shown in health assessment 100, some patient data (patient data 108) for a set of biomarkers 102 is acquired at one or more particular time points within period of time 112. Patient data 108 may be acquired, for example, at the time of an annual physical or a hospital visit of the patient. However, some patient data (missing data 110) is missing for one or more biomarkers 102 and/or one or more particular time points within period of time 112. The particular time points are represented as time embeddings 104 defining the relative temporal position of patient data 108 and missing data 110. At times, diagnoses and/or procedures are also performed, represented as diagnosis or procedure codes 106, based on patient data 108. Missing data 110 posits a significant challenge to the development and training of conventional AI (artificial intelligence) based models for performing medical analysis tasks, as such conventional AI based models tend to overfit to such missing data.

Embodiments described herein provide for a transformer-based deep learning model for clinical decision support to generate recommendations for a clinical course of action by implicitly imputing missing data 110 as a means for regularizing the model using available context information. Embodiments described herein thus address the problem of high sensitivity and specificity experienced by conventional AI based models. Further, embodiments described herein present the risk scores to a user to facilitate user interpretation and adoption. Advantageously, contrary to conventional AI based models, embodiments described herein provide for clinical decision support, for example, by determining a risk score of one or more cancers, without the need to a priori impute missing data 110.

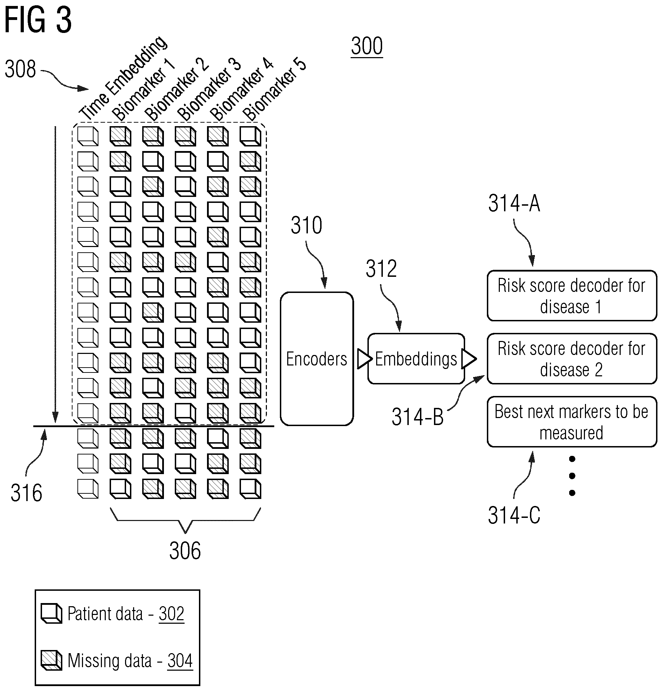

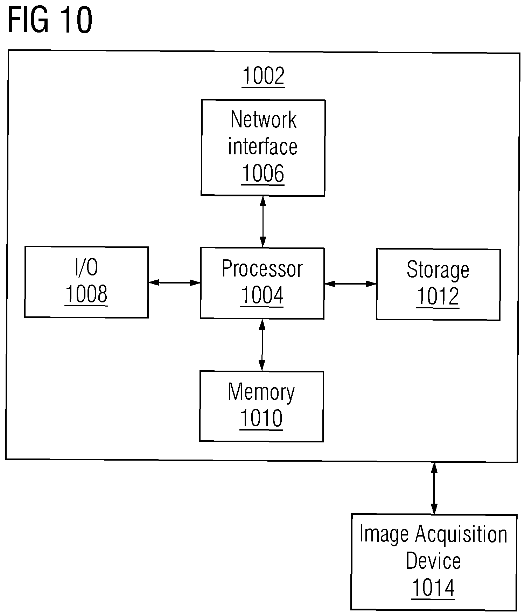

Figure 2 shows a method 200 for performing one or more medical analysis tasks for clinical decision support, in accordance with one or more embodiments. The steps of method 200 may be performed by one or more suitable computing devices, such as, e.g., computer 1002 of Figure 10. Figure 3 shows an architectural diagram 300 of an AI based system for performing one or more medical analysis tasks for clinical decision support, in accordance with one or more embodiments. Figure 2 and Figure 3 will be described together.

At step 202 of Figure 2, patient data of a patient for a set of biomarkers acquired at one or more time points within a period of time is received. As used herein, a biomarker refers to any measurable characteristic of the patient. Examples of biomarkers of the plurality of biomarkers include vital signs (e.g., heart rate, body temperature, respiratory rate, blood oxygen saturation levels, etc.), laboratory measurements (both current and historical), demographic information (e.g., age, sex), family history, measurements and other information derived from medical images, genetic biomarkers of the patient, and embeddings, risk scores, or other outputs generated by a machine learning based system. However, the plurality of biomarkers may include any other suitable biomarker of the patient. The biomarkers may be encoded with time embeddings defining a relative temporal position relative to a given reference time.

In one embodiment, the patient data is missing data for certain biomarkers of the set of biomarkers for certain time points within the period of time. In one example, as shown in Figure 1, the patient data is patient data 108 in health assessment 100 and the missing data is missing data 110 in health assessment 100. In another example, as shown in architectural diagram 300 of Figure 3, the patient data is patient data 302 of a set of biomarkers 306 acquired at various time points, represented as time embeddings 308 defining a relative temporal positioning relative to a given reference time 316, and the missing data is missing data 304.

The patient data may be received by loading previously acquired patient data from a storage or memory of a computer system or receiving patient data that have been transmitted from a remote computer system. In one embodiment, the patient data may be received from a computing device (e.g., computer 1002 of Figure 10), such as, e.g., a mobile phone, tablet, computer, etc., from which the patient is interacting.

At step 204 of Figure 2, the patient data is encoded using an encoder network to generate patient data embeddings. In one example, as shown in architectural diagram 300 of Figure 3, included patient data 302 of set of biomarkers 306 is encoded by encoder 310 to generate embeddings 312.

In one embodiment, the encoder network is a transformer-based encoder network. However, the encoder network may be implemented in accordance with any other suitable machine learning based architecture. The encoder network receives as input the patient data encoded with the time embeddings and generates as output the patient data embeddings. The encoder network thereby maps the sparse patient data to an information bottleneck (i.e., the embeddings). The patient data embeddings may be represented as a vector representing a low-level latent encoding of the patient data of the patient with respect to a particular disease. In one embodiment, the patient data embeddings are regularized to be a Gaussian model, such as, e.g., a Gaussian VAE (variational autoencoder) model or a Gaussian mixture model (e.g., Dirichlet model).

At step 206 of Figure 2, one or more medical analysis tasks are performed based on the patient data embeddings using one or more decoder networks. The one or more medical analysis tasks comprise generating recommendations for a clinical course of action. For example, the recommendations for the clinical course of action may include ordering tests or procedures for acquiring additional patient data for one or more biomarkers to increase confidence in the model output for a given medical condition or performing additional medical procedures to further aid the diagnosis. In one example, as shown in architectural diagram 300 of Figure 3, the one or more decoder networks are decoder networks 314-A, 314-B, and 314-C (collectively referred to as decoder networks 314) for decoding embeddings 312 to respectively perform the medical analysis tasks of determining a risk score for disease 1, determining a risk score for disease 2, and determining a best next biomarker to be measured.

In one embodiment, for example wherein the patient data is missing data for certain biomarkers of the set of biomarkers for certain time points within the period of time, the one or more medical analysis tasks are performed using the one or more decoder networks by imputing the missing data for the certain biomarkers for the certain time points. The one or more decoder networks implicitly impute the missing data and may or may not output the imputed missing data.

In one embodiment, the one or more decoder networks are one or more transformer-based decoder networks. The transformer-based decoder networks learn to implicitly impute the missing data from the patient data embeddings during the training stage. However, the decoder network may be implemented in accordance with any other suitable machine learning based architecture. Each of the one or more decoder networks receives as input the patient data embeddings and generates as output results of a respective medical analysis task. The results may be for a continuous time periods (e.g., 12 to 24 months) or after a fixed time interval (e.g., 24 hours).

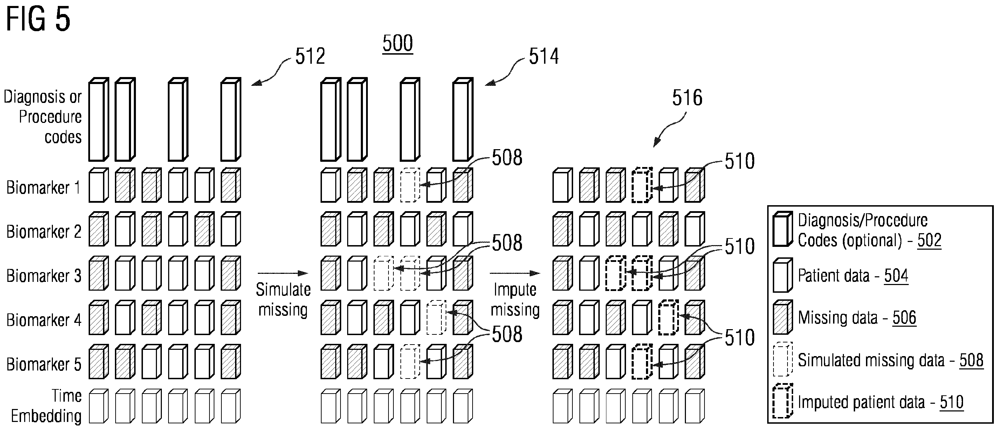

In one embodiment, the one or more medical analysis tasks comprise tasks for clinical decision support, such as, e.g., determining a time to discharge of the patient, determining a survival score (i.e., time to fatality) of the patient, determining a health status of various organs and organ systems of the patient, determining a risk score associated with the likelihood of the patient developing a disease or other medical condition within a certain time interval, risk stratifying patients for a disease or a set of diseases (e.g., cancer) in order to identify the population that can identify patients who should be immediately screened, generating a confidence interval associated with another one of the one or more medical analysis tasks. The disease may be, for example, a type of cancer (e.g., brain, head/neck, thyroid, lung, liver, colon, rectal, etc.). In one embodiment, the one or more decoders may be Gaussian process decoders that output a risk score for the disease and a confidence interval associated with the risk score. The confidence interval may be represented, e.g., categorically (e.g., low, medium, or high risk) or as a score. In one embodiment, the one or more medical analysis tasks comprise tasks for regularizing model training, such as, e.g., explicitly imputing patient data simulated as being missing data (as described with respect to Figure 5).

At step 208 of Figure 2, results of the one or more medical analysis tasks are output. For example, the results of the one or more medical analysis tasks can be output by displaying the results on a display device of a computer system (e.g., computer 1002 of Figure 10), storing the results on a memory or storage of a computer system, or by transmitting the results to a remote computer system.

In one embodiment, the results of the one or more medical analysis tasks are output as a health report. The health report may be an enriched lab report indicating, for example, the disease status, risk scores, pre- and post-test likelihood, and a likelihood ratio. The health report may be displayed to a user (e.g., the patient or a clinician) via a display device on, e.g., a phone, tablet, computer, etc. The health report may include recommendations for additional clinical measurements or procedures (e.g., lab work).

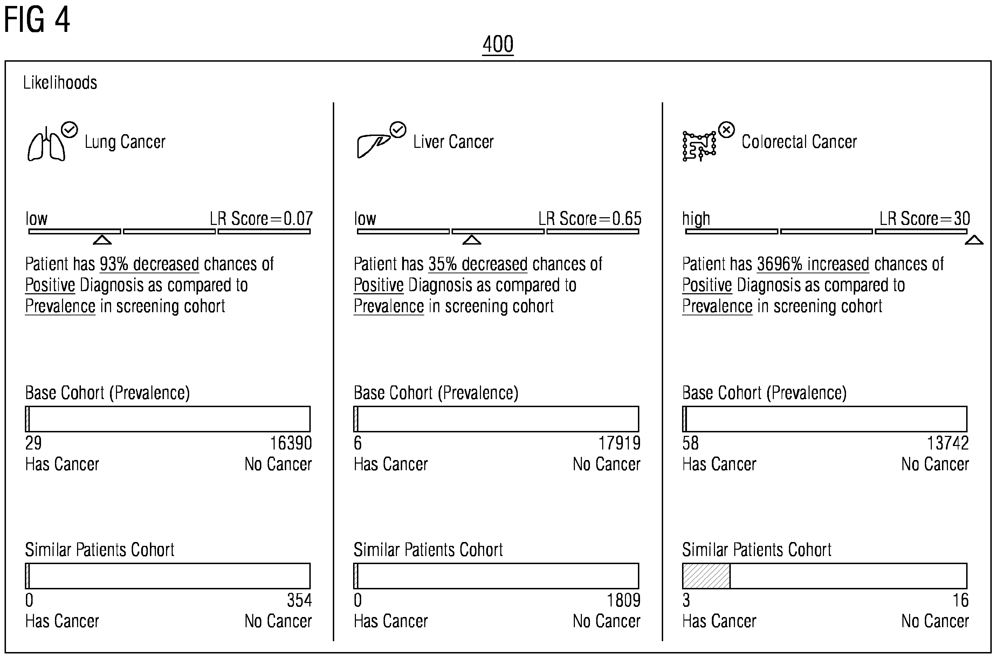

Figure 4 shows a user interface 400 presenting results of the one or more medical analysis tasks, in accordance with one or more embodiments. User interface 400 may be presented at step 208 of Figure 2 for outputting the results of the one or more medical analysis tasks. User interface 400 presents a LR (likelihood ratio) score, a base cohort, and a similar patients cohort. The LR score is computed as a ratio of the pre-test likelihood and the post-test likelihood of the patient having a particular disease. The pre-test likelihood refers to the likelihood of the patients having the disease prior to a test being performed (i.e., assigning a likelihood based on whatever information was known about the patient prior to the test). Post-test likelihood refers to the likelihood of the patients having the disease based on results of the test. For instance, if the test results in a low score, it is very likely the patient may not have the disease/medical condition.

For example, consider a base cohort of 1000 patients with 4 patients that developed colorectal cancer (CRC) within 1 year. The pre-test probability of a patient from this population is 0.4%. Consider 1 patient and using the model to compute the risk profile of the patient (score S with confidence interval CI), all patients with similar risk profile (i.e., risk scores within the range of S-CI and S+CI) are identified. The similar patients are referred to as a "similar patients cohort" since they have similar risk profiles. Without loss of generality, the size of the similar patients cohort is 50 patients including the 4 patients who developed CRC. Then the post-test probability of the patient is 4/50=8%. Now the LR ratio of the patients is therefore 8/0.4=20.

The LR score may be computed for each particular disease. The particular diseases shown in user interface 400 are lung cancer, liver cancer, and colorectal cancer. The base cohort is determined as a general randomized sampling of patients. The risk score and the confidence interval are used to identify the subgroup of patients in the base cohort with similar scores, thus resulting in the similar patients cohort (i.e., a patient cohort with similar risk scores).

In one embodiment, the encoder network (utilized at step 204 of Figure 2) and the one or more decoder networks (utilized at step 206 of Figure 2) are trained during a prior offline or training stage using training patient data for a large cohort of patients. Each of the one or more decoder networks are trained according to a respective loss function for performing a particular medical analysis task. In one embodiment, the one or more decoder networks are trained for imputing missing data according to Figure 5. Once trained, the encoder network and the one or more decoder networks are applied during an online or inference stage, e.g., to respectively perform steps 204 and 206 of Figure 2.

Figure 5 shows a workflow 500 for training a decoder network for imputing missing data, in accordance with one or more embodiments. In workflow 500, training patient data 512 is provided. Training patient data 512 includes patient data 504 and diagnosis or procedure codes 502 (optional) acquired at various time embeddings. Training patient data 512 does not include missing data 506 for certain biomarkers for certain time embeddings. Simulated training patient data 514 is generated by removing data (simulated missing data 508) from patient data 504, thereby simulating certain patient data 504 as being missing in patient data 504. The decoder network is trained to generate imputed training patient data 516 by imputing simulated missing data 508 to determine imputed patient data 510.

In one embodiment, the encoder network (utilized at step 204 of Figure 2) and the one or more decoder networks (utilized at step 206 of Figure 2) may form an ensemble of machine learning based networks. The outputs generated by the decoder networks are generated as an aggregate from the ensemble. This would reduce undesirable biases in the data that could otherwise be present in a single network. Given a multitude of networks, a confidence interval for the risk score can be computed. Each network is trained on different subsets of the training data, accounting for variations in the acquisition protocols across hospitals, availability of various biomarkers, measurement methods used to assess the biomarkers, as well as biomarker measurements such as, e.g., laboratory values, age range, comorbidities, etc.

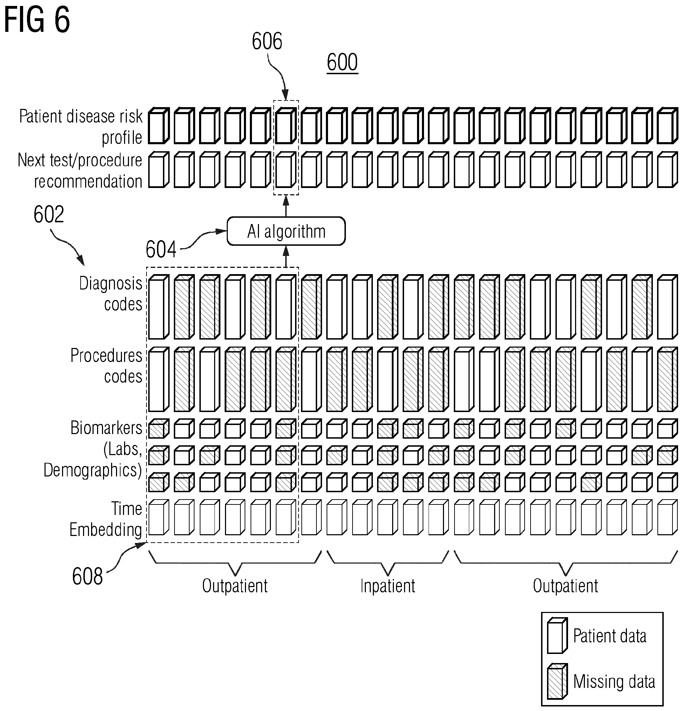

In one embodiment, the encoder network (utilized at step 204 of Figure 2) and the one or more decoder networks (utilized at step 206 of Figure 2) are trained using deep reinforcement learning. Figure 6 shows a workflow 600 for training an encoder network and one or more decoder networks for performing one or more medical analysis tasks for clinical decision support using deep reinforcement learning, in accordance with one or more embodiments. Workflow 600 shows longitudinal training data 602 comprising training patient data of biomarkers, procedure codes, and diagnosis codes acquired at various time points. The time points are represented as time embeddings in workflow 600 defining relative temporal positions. Training data 602 includes outpatient and inpatient data.

AI algorithm 604 is trained to generate output 606 using deep reinforcement learning based on training data 602 within time window 608. Output 606 comprises a patient disease risk profile and a recommendation for performing a next test/procedure. In one embodiment, AI algorithm 604 comprises the encoder network utilized at step 204 of Figure 2 and the one or more decoder networks utilized at step 206 of Figure 2. During training, time window 608 continuously shifts to iterate over training data 602.

In deep reinforcement learning, an AI agent navigates through an environment defined by training patient data 602 by taking one or more actions to generate a recommendation for a next test or procedure to be performed to thereby acquire patient data for a biomarker or a procedure. A reward is provided to the AI agent if the recommendation helps in the diagnosis. The policy network imputes the missing training patient data using the transformer-based decoder networks and the time of the current encounter as the time embedding.

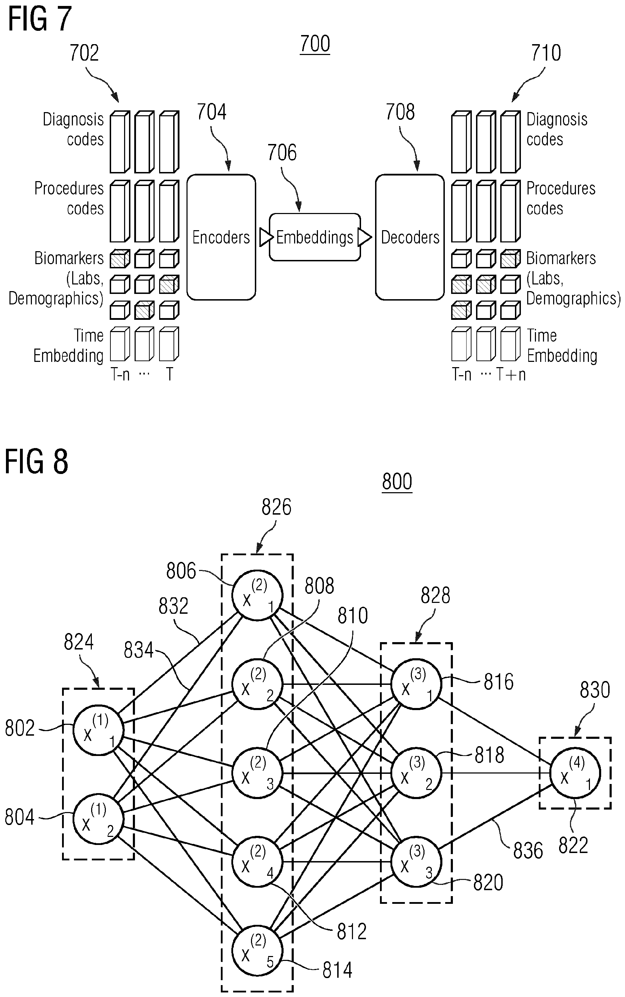

One challenge is that training patient data 602 does not include data on the outcome of procedures that were not performed. To address this challenge, training data 602 comprises future patient data estimated by a machine learning based state transition model. An exemplary network architecture for the state transition model is shown in Figure 7.

Figure 7 shows a network architecture 700 for a state transition model for estimating future patient data, in accordance with one or more embodiments. Encoder network 704 receives as input patient data 702 and encodes patient data 702 into embeddings 706. Patient data 702 comprises patient data from a time T-n to a time T. Decoder network 708 receives as input embeddings 706 and decodes embeddings 706 to generate as output future patient data 708. Future patient data 708 comprises predicted patient data 710 for a time T-n to a time T+n.

Referring back to Figure 6, the state transition model is trained a priori to the training of AI algorithm 604. To incorporate the state transition model, the standard deep reinforcement learning approach is adjusted. In standard deep reinforcement learning, while state transitions can be stochastic, when an AI agent takes an action to modify the environment, the subsequent state, once determined, is unambiguous. However, in the environment defined by training patient data 602, when an action is performed to acquire patient data for a procedure that is different from the procedure actually performed in training patient data 602, the environment cannot determine the state unambiguously. In one embodiment, the state transition model is utilized to determine the future states. If the AI agent recommends obtaining patient data for certain biomarkers, the state transition model is utilized to estimate patient data for biomarkers which is not included in training patient data 602. This process continues until the end of the episode (i.e., the sequence of biomarkers for which patient data is to be acquired is determined). If the diagnosis obtained is the diagnosis performed in training patient data 602, the AI agent receives a reward.

A reward is provided to the AI agent at the end of every episode if a correct diagnosis is made. For every encounter, the AI agent incurs a penalty that encourages the AI agent to obtain he diagnosis early. Training patient data 602 includes procedures that were performed that resulted in a negative diagnosis. Thus, incurring a small penalty for every encounter encourages the AI agent to be able to make the diagnosis early, while still requiring sufficient confidence in the diagnosis. This implicitly encourages the AI agent to identify patients at risk early.

In one embodiment, the deep reinforcement learning approach in accordance with embodiments described herein may be extended to optimize the diagnostic process to be cost sensitive, where a cost is associated with ordering each biomarker which is representative of the actual cost of the test.

In one embodiment, the deep reinforcement learning approach in accordance with embodiments described herein may be extended to reward the AI agent if it follows established clinical guidelines based on the symptoms of the patient.

In one embodiment, AI algorithm 604 may be refined after deployed in the field. AI algorithm 604 may be deployed as a companion to clinicians. The AI agent presents recommendations to the clinicians, who can then override the recommendations. This information is used to update the AI agent. Subsequently, supervision may be reduced by ordering the biomarker tests recommended by the AI agent and augmenting the recommendations with additional tests the clinicians may be interested in. This can then be continued to further reduce supervision on non-invasive procedures, such as, e.g., ultrasound/MRI (magnetic resonance imaging), followed by x-ray, CT (computed tomography), etc.

In one embodiment, the deep reinforcement learning approach in accordance with embodiments described herein may be extended for patient treatment and management. In such a setting, the state transition network takes medication into account and recommends therapy procedures as well as medications to treat the patient.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based models, as well as with respect to methods and systems for training machine learning based models. Features, advantages or alternative embodiments herein can be assigned to the other claimed objects and vice versa. In other words, claims for methods and systems for training a machine learning based model can be improved with features described or claimed in context of the methods and systems for utilizing a trained machine learning based model, and vice versa.

In particular, the trained machine learning based models applied in embodiments described herein can be adapted by the methods and systems for training the machine learning based models. Furthermore, the input data of the trained machine learning based model can comprise advantageous features and embodiments of the training input data, and vice versa. Furthermore, the output data of the trained machine learning based model can comprise advantageous features and embodiments of the output training data, and vice versa.

In general, a trained machine learning based model mimics cognitive functions that humans associate with other human minds. In particular, by training based on training data, the trained machine learning based model is able to adapt to new circumstances and to detect and extrapolate patterns.

In general, parameters of a machine learning based model can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based model can be adapted iteratively by several steps of training.

In particular, a trained machine learning based model can comprise a neural network, a support vector machine, a decision tree, and/or a Bayesian network, and/or the trained machine learning based model can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

Figure 8 shows an embodiment of an artificial neural network 800, in accordance with one or more embodiments. Alternative terms for "artificial neural network" are "neural network", "artificial neural net" or "neural net". Machine learning networks described herein, such as, e.g., the encoder network utilized at step 204 and the one or more decoder networks utilized at step 206 of Figure 2, encoders 310 and decoders 314 of Figure 3, AI algorithm 604 of Figure 6, and encoder network 704 and decoder network 708 of Figure 7, may be implemented using artificial neural network 800.

The artificial neural network 800 comprises nodes 802-822 and edges 832, 834, ..., 836, wherein each edge 832, 834, ..., 836 is a directed connection from a first node 802-822 to a second node 802-822. In general, the first node 802-822 and the second node 802-822 are different nodes 802-822, it is also possible that the first node 802-822 and the second node 802-822 are identical. For example, in Figure 8, the edge 832 is a directed connection from the node 802 to the node 806, and the edge 834 is a directed connection from the node 804 to the node 806. An edge 832, 834, ..., 836 from a first node 802-822 to a second node 802-822 is also denoted as "ingoing edge" for the second node 802-822 and as "outgoing edge" for the first node 802-822.

In this embodiment, the nodes 802-822 of the artificial neural network 800 can be arranged in layers 824-830, wherein the layers can comprise an intrinsic order introduced by the edges 832, 834, ..., 836 between the nodes 802-822. In particular, edges 832, 834, ..., 836 can exist only between neighboring layers of nodes. In the embodiment shown in Figure 8, there is an input layer 824 comprising only nodes 802 and 804 without an incoming edge, an output layer 830 comprising only node 822 without outgoing edges, and hidden layers 826, 828 in-between the input layer 824 and the output layer 830. In general, the number of hidden layers 826, 828 can be chosen arbitrarily. The number of nodes 802 and 804 within the input layer 824 usually relates to the number of input values of the neural network 800, and the number of nodes 822 within the output layer 830 usually relates to the number of output values of the neural network 800.

In particular, a (real) number can be assigned as a value to every node 802-822 of the neural network 800. Here, $x^{(n)}_i$ denotes the value of the i-th node 802-822 of the n-th layer 824-830. The values of the nodes 802-822 of the input layer 824 are equivalent to the input values of the neural network 800, the value of the node 822 of the output layer 830 is equivalent to the output value of the neural network 800. Furthermore, each edge 832, 834, ..., 836 can comprise a weight being a real number, in particular, the weight is a real number within the interval [-1, 1] or within the interval [0, 1]. Here, $w^{(m,n)}_{i,j}$ denotes the weight of the edge between the i-th node 802-822 of the m-th layer 824-830 and the j-th node 802-822 of the n-th layer 824-830. Furthermore, the abbreviation $w^{(n)}_{i,j}$ is defined for the weight $w^{(n,n+1)}_{i,j}$.

In particular, to calculate the output values of the neural network 800, the input values are propagated through the neural network. In particular, the values of the nodes 802-822 of the (n+1)-th layer 824-830 can be calculated based on the values of the nodes 802-822 of the n-th layer 824-830 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 824 are given by the input of the neural network 800, wherein values of the first hidden layer 826 can be calculated based on the values of the input layer 824 of the neural network, wherein values of the second hidden layer 828 can be calculated based in the values of the first hidden layer 826, etc.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 800 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta_j^{(n)} = \left( \sum_k \delta_k^{(n+1)} \cdot w_{j,k}^{(n+1)} \right) \cdot f' \left( \sum_i x_i^{(n)} \cdot w_{i,j}^{(n)} \right)$$

based on $\delta^{(n+1)}{}_j$, if the (n+1)-th layer is not the output layer, and

$$\delta_j^{(n)} = \left( x_k^{(n+1)} \cdot t_j^{(n+1)} \right) \cdot f' \left( \sum_i x_i^{(n)} \cdot w_{i,j}^{(n)} \right)$$

if the (n+1)-th layer is the output layer 830, wherein f' is the first derivative of the activation function, and $y^{(n+1)}{}_j$ is the comparison training value for the j-th node of the output layer 830.

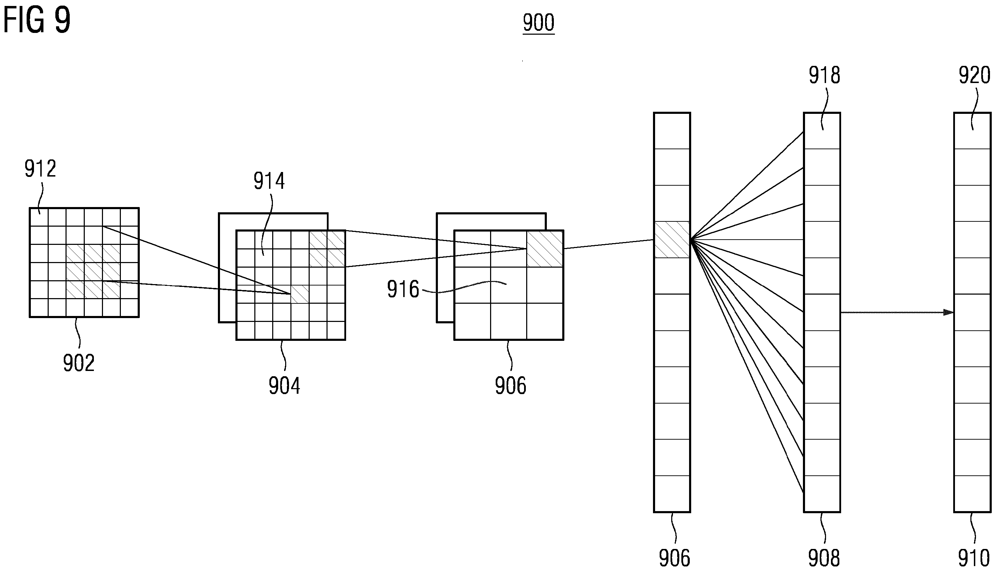

Figure 9 shows a convolutional neural network 900, in accordance with one or more embodiments. Machine learning networks described herein, such as, e.g., the encoder network utilized at step 204 and the one or more decoder networks utilized at step 206 of Figure 2, encoders 310 and decoders 314 of Figure 3, AI algorithm 604 of Figure 6, and encoder network 704 and decoder network 708 of Figure 7, may be implemented using convolutional neural network 900.

In the embodiment shown in Figure 9, the convolutional neural network comprises 900 an input layer 902, a convolutional layer 904, a pooling layer 906, a fully connected layer 908, and an output layer 910. Alternatively, the convolutional neural network 900 can comprise several convolutional layers 904, several pooling layers 906, and several fully connected layers 908, as well as other types of layers. The order of the layers can be chosen arbitrarily, usually fully connected layers 908 are used as the last layers before the output layer 910.

In particular, within a convolutional neural network 900, the nodes 912-920 of one layer 902-910 can be considered to be arranged as a d-dimensional matrix or as a d-dimensional image. In particular, in the two-dimensional case the value of the node 912-920 indexed with i and j in the n-th layer 902-910 can be denoted as $x^{(n)}{}_{[i,j]}$. However, the arrangement of the nodes 912-920 of one layer 902-910 does not have an effect on the calculations executed within the convolutional neural network 900 as such, since these are given solely by the structure and the weights of the edges.

In particular, a convolutional layer 904 is characterized by the structure and the weights of the incoming edges forming a convolution operation based on a certain number of kernels. In particular, the structure and the weights of the incoming edges are chosen such that the values $x^{(n)}{}_k$ of the nodes 914 of the convolutional layer 904 are calculated as a convolution $x^{(n)}{}_k = K_k * x^{(n-1)}$ based on the values $x^{(n-1)}$ of the nodes 912 of the preceding layer 902, where the convolution * is defined in the two-dimensional case as

$$x_k^{(n)}[i,j] = \left( K_k * x^{(n-1)} \right)[i,j] = \sum_{i'} \sum_{j'} K_k[i',j'] \cdot x^{(n-1)}[i-i', j-j'] \, .$$

Here the k-th kernel $K_k$ is a d-dimensional matrix (in this embodiment a two-dimensional matrix), which is usually small compared to the number of nodes 912-918 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 912-920 in the respective layer 902-910. in particular, for a convolutional layer 904, the number of nodes 914 in the convolutional layer is equivalent to the number of nodes 912 in the preceding layer 902 multiplied with the number of kernels.

If the nodes 912 of the preceding layer 902 are arranged as a d-dimensional matrix, using a plurality of kernels can be interpreted as adding a further dimension (denoted as "depth" dimension), so that the nodes 914 of the convolutional layer 904 are arranged as a (d+1)-dimensional matrix. If the nodes 912 of the preceding layer 902 are already arranged as a (d+1)-dimensional matrix comprising a depth dimension, using a plurality of kernels can be interpreted as expanding along the depth dimension, so that the nodes 914 of the convolutional layer 904 are arranged also as a (d+1)-dimensional matrix, wherein the size of the (d+1)-dimensional matrix with respect to the depth dimension is by a factor of the number of kernels larger than in the preceding layer 902.

The advantage of using convolutional layers 904 is that spatially local correlation of the input data can exploited by enforcing a local connectivity pattern between nodes of adjacent layers, in particular by each node being connected to only a small region of the nodes of the preceding layer.

In embodiment shown in Figure 9, the input layer 902 comprises 36 nodes 912, arranged as a two-dimensional 6x6 matrix. The convolutional layer 904 comprises 72 nodes 914, arranged as two two-dimensional 6x6 matrices, each of the two matrices being the result of a convolution of the values of the input layer with a kernel. Equivalently, the nodes 914 of the convolutional layer 904 can be interpreted as arranges as a three-dimensional 6x6x2 matrix, wherein the last dimension is the depth dimension.

A pooling layer 906 can be characterized by the structure and the weights of the incoming edges and the activation function of its nodes 916 forming a pooling operation based on a non-linear pooling function f. For example, in the two dimensional case the values $x^{(n)}$ of the nodes 916 of the pooling layer 906 can be calculated based on the values $x^{(n-1)}$ of the nodes 914 of the preceding layer 904 as

$$x^{(n)}[i,j] = f(x^{(n-1)}[id_1, jd_2], ..., x^{(n-1)}[id_1+d_1-1, jd_2+d_2-1])$$

In other words, by using a pooling layer 906, the number of nodes 914, 916 can be reduced, by replacing a number d1 d2 of neighboring nodes 914 in the preceding layer 904 with a single node 916 being calculated as a function of the values of said number of neighboring nodes in the pooling layer. In particular, the pooling function f can be the max-function, the average or the L2-Norm. In particular, for a pooling layer 906 the weights of the incoming edges are fixed and are not modified by training.

The advantage of using a pooling layer 906 is that the number of nodes 914, 916 and the number of parameters is reduced. This leads to the amount of computation in the network being reduced and to a control of overfitting.

In the embodiment shown in Figure 9, the pooling layer 906 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 908 can be characterized by the fact that a majority, in particular, all edges between nodes 916 of the previous layer 906 and the nodes 918 of the fully-connected layer 908 are present, and wherein the weight of each of the edges can be adjusted individually.

In this embodiment, the nodes 916 of the preceding layer 906 of the fully-connected layer 908 are displayed both as two-dimensional matrices, and additionally as non-related nodes (indicated as a line of nodes, wherein the number of nodes was reduced for a better presentability). In this embodiment, the number of nodes 918 in the fully connected layer 908 is equal to the number of nodes 916 in the preceding layer 906. Alternatively, the number of nodes 916, 918 can differ.

Furthermore, in this embodiment, the values of the nodes 920 of the output layer 910 are determined by applying the Softmax function onto the values of the nodes 918 of the preceding layer 908. By applying the Softmax function, the sum the values of all nodes 920 of the output layer 910 is 1, and all values of all nodes 920 of the output layer are real numbers between 0 and 1.

A convolutional neural network 900 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 900 can be trained based on the backpropagation algorithm. For preventing overfitting, methods of regularization can be used, e.g. dropout of nodes 912-920, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 2-3 and 5-7. Certain steps or functions of the methods and workflows described herein, including one or more of the steps or functions of Figures 2-3 and 5-7, may be performed by a server or by another processor in a network-based cloud-computing system. Certain steps or functions of the methods and workflows described herein, including one or more of the steps of Figures 2-3 and 5-7, may be performed by a client computer in a network-based cloud computing system. The steps or functions of the methods and workflows described herein, including one or more of the steps of Figures 2-3 and 5-7, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 2-3 and 5-7, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

A high-level block diagram of an example computer 1002 that may be used to implement systems, apparatus, and methods described herein is depicted in Figure 10. Computer 1002 includes a processor 1004 operatively coupled to a data storage device 1012 and a memory 1010. Processor 1004 controls the overall operation of computer 1002 by executing computer program instructions that define such operations. The computer program instructions may be stored in data storage device 1012, or other computer readable medium, and loaded into memory 1010 when execution of the computer program instructions is desired. Thus, the method and workflow steps or functions of Figures 2-3 and 5-7 can be defined by the computer program instructions stored in memory 1010 and/or data storage device 1012 and controlled by processor 1004 executing the computer program instructions. For example, the computer program instructions can be implemented as computer executable code programmed by one skilled in the art to perform the method and workflow steps or functions of Figures 2-3 and 5-7. Accordingly, by executing the computer program instructions, the processor 1004 executes the method and workflow steps or functions of Figures 2-3 and 5-7. Computer 1002 may also include one or more network interfaces 1006 for communicating with other devices via a network. Computer 1002 may also include one or more input/output devices 1008 that enable user interaction with computer 1002 (e.g., display, keyboard, mouse, speakers, buttons, etc.).

Processor 1004 may include both general and special purpose microprocessors, and may be the sole processor or one of multiple processors of computer 1002. Processor 1004 may include one or more central processing units (CPUs), for example. Processor 1004, data storage device 1012, and/or memory 1010 may include, be supplemented by, or incorporated in, one or more application-specific integrated circuits (ASICs) and/or one or more field programmable gate arrays (FPGAs).

Data storage device 1012 and memory 1010 each include a tangible non-transitory computer readable storage medium. Data storage device 1012, and memory 1010, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

input/output devices 1008 may include peripherals, such as a printer, scanner, display screen, etc. For example, input/output devices 1008 may include a display device such as a cathode ray tube (CRT) or liquid crystal display (LCD) monitor for displaying information to the user, a keyboard, and a pointing device such as a mouse or a trackball by which the user can provide input to computer 1002.

An image acquisition device 1014 can be connected to the computer 1002 to input image data (e.g., medical images) to the computer 1002. it is possible to implement the image acquisition device 1014 and the computer 1002 as one device. It is also possible that the image acquisition device 1014 and the computer 1002 communicate wirelessly through a network. In a possible embodiment, the computer 1002 can be located remotely with respect to the image acquisition device 1014.

Any or all of the systems and apparatus discussed herein may be implemented using one or more computers such as computer 1002.

One skilled in the art will recognize that an implementation of an actual computer or computer system may have other structures and may contain other components as well, and that Figure 10 is a high level representation of some of the components of such a computer for illustrative purposes.

Independent of the grammatical term usage, individuals with male, female or other gender identities are included within the term.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope and spirit of the invention. Those skilled in the art could implement various other feature combinations without departing from the scope and spirit of the invention.

The following is a list of non-limiting illustrative embodiments disclosed herein:

Illustrative embodiment 1. A computer-implemented method comprising: receiving patient data of a patient for a set of biomarkers acquired at one or more time points within a period of time,; encoding the patient data using an encoder network to generate patient data embeddings; performing one or more medical analysis tasks based on the patient data embeddings using one or more decoder networks, the one or more medical analysis tasks comprising generating recommendations for a clinical course of action; and outputting results of the one or more medical analysis tasks.

Illustrative embodiment 2. The computer-implemented method of illustrative embodiment 1, wherein the patient data is missing data for certain biomarkers of the set of biomarkers for certain time points within the period of time, and performing one or more medical analysis tasks based on the patient data embeddings using one or more decoder networks comprises: imputing the missing data for the certain biomarkers for the certain time points.

Illustrative embodiment 3. The computer-implemented method according to one of the preceding embodiments, wherein the patient data is encoded with time embeddings defining a relative temporal position relative to a given reference time, and encoding the patient data using an encoder network to generate patient data embeddings comprises: encoding the patient data encoded with the time embeddings using the encoder network to generate the patient data embeddings.

Illustrative embodiment 4. The computer-implemented method according to one of the preceding embodiments, wherein the encoder network comprises a transformer-based encoder network and the one or more decoder networks comprise one or more transformer-based decoder networks.

Illustrative embodiment 5. The computer-implemented method according to one of the preceding embodiments, wherein the one or more medical analysis tasks comprise determining a risk score associated with a disease.

Illustrative embodiment 6. The computer-implemented method according to one of the preceding embodiments, wherein the one or more medical analysis tasks comprise determining a confidence interval associated with another task of the one or more medical analysis tasks.

Illustrative embodiment 7. The computer-implemented method according to one of the preceding embodiments, wherein outputting results of the one or more medical analysis tasks comprises: presenting a user interface depicting a likelihood ratio score and a cohort of patients similar to the patient.

Illustrative embodiment 8. The computer-implemented method according to one of the preceding embodiments, wherein the encoder network and the one or more decoder networks are trained by simulating certain data for one or more biomarkers of the set of biomarkers as being missing in training patient data.

Illustrative embodiment 9. The computer-implemented method according to one of the preceding embodiments, wherein the encoder network and the one or more decoder networks are trained using deep reinforcement learning by iterating over a window of training patient data and estimated future patient data.

Illustrative embodiment 10. An apparatus comprising: means for receiving patient data of a patient for a set of biomarkers acquired at one or more time points within a period of time; means for encoding the patient data using an encoder network to generate patient data embeddings; means for performing one or more medical analysis tasks based on the patient data embeddings using one or more decoder networks, the one or more medical analysis tasks comprising generating recommendations for a clinical course of action; and means for outputting results of the one or more medical analysis tasks.

Illustrative embodiment 11. The apparatus of illustrative embodiment 10, wherein the patient data is missing data for certain biomarkers of the set of biomarkers for certain time points within the period of time, and the means for performing one or more medical analysis tasks based on the patient data embeddings using one or more decoder networks comprises: means for imputing the missing data for the certain biomarkers for the certain time points.

Illustrative embodiment 12. The apparatus of any one of illustrative embodiments 10-11, wherein the patient data is encoded with time embeddings defining a relative temporal position relative to a given reference time, and the means for encoding the patient data using an encoder network to generate patient data embeddings comprises: means for encoding the patient data encoded with the time embeddings using the encoder network to generate the patient data embeddings.

Illustrative embodiment 13. The apparatus of any one of illustrative embodiments 10-12, wherein the encoder network comprises a transformer-based encoder network and the one or more decoder networks comprise one or more transformer-based decoder networks.

Illustrative embodiment 14. The apparatus of any one of illustrative embodiments 10-13, wherein the one or more medical analysis tasks comprise determining a risk score associated with a disease.

Illustrative embodiment 15. A non-transitory computer readable medium storing computer program instructions, the computer program instructions when executed by a processor cause the processor to perform operations comprising: receiving patient data of a patient for a set of biomarkers acquired at one or more time points within a period of time; encoding the patient data using an encoder network to generate patient data embeddings; performing one or more medical analysis tasks based on the patient data embeddings using one or more decoder networks, the one or more medical analysis tasks comprising generating recommendations for a clinical course of action; and outputting results of the one or more medical analysis tasks.

Illustrative embodiment 16. The non-transitory computer readable medium of illustrative embodiment 15, wherein the patient data is missing data for certain biomarkers of the set of biomarkers for certain time points within the period of time, and performing one or more medical analysis tasks based on the patient data embeddings using one or more decoder networks comprises: imputing the missing data for the certain biomarkers for the certain time points.

Illustrative embodiment 17. The non-transitory computer readable medium of any one of illustrative embodiments 15-16, wherein the one or more medical analysis tasks comprise determining a confidence interval associated with another task of the one or more medical analysis tasks.

Illustrative embodiment 18. The non-transitory computer readable medium of any one of illustrative embodiments 15-17, wherein outputting results of the one or more medical analysis tasks comprises: presenting a user interface depicting a likelihood ratio score and a cohort of patients similar to the patient.

Illustrative embodiment 19. The non-transitory computer readable medium of any one of illustrative embodiments 15-18, wherein the encoder network and the one or more decoder networks are trained by simulating certain data for one or more biomarkers of the set of biomarkers as being missing in training patient data.

Illustrative embodiment 20. The non-transitory computer readable medium of any one of illustrative embodiments 15-19, wherein the encoder network and the one or more decoder networks are trained using deep reinforcement learning by iterating over a window of training patient data and estimated future patient data.

**Claims**

1. A computer-implemented method comprising:

   receiving (202) patient data (108, 302) of a patient for a set of biomarkers (102, 306) acquired at one or more time points within a period of time (112);
   encoding (204) the patient data using an encoder network (310) to generate patient data embeddings (312);
   performing (206) one or more medical analysis tasks based on the patient data embeddings using one or more decoder networks (314), the one or more medical analysis tasks comprising generating recommendations for a clinical course of action; and
   outputting (208) results of the one or more medical analysis tasks.

2. The computer-implemented method of claim 1, wherein the patient data is missing data (110, 304) for certain biomarkers of the set of biomarkers for certain time points within the period of time, and performing one or more medical analysis tasks based on the patient data embeddings using one or more decoder networks comprises: imputing the missing data for the certain biomarkers for the certain time points.

3. The computer-implemented method of claim 1 or 2, wherein the patient data is encoded with time embeddings (104, 308) defining a relative temporal position relative to a given reference time (316), and encoding the patient data using an encoder network to generate patient data embeddings comprises:
   encoding the patient data encoded with the time embeddings using the encoder network to generate the patient data embeddings.

4. The computer-implemented method of claim 1, 2 or 3, wherein the encoder network comprises a transformer-based encoder network and the one or more decoder networks comprise one or more transformer-based decoder networks.

5. The computer-implemented method of one of claims 1-4, wherein the one or more medical analysis tasks comprise determining a risk score associated with a disease.

6. The computer-implemented method of one of claims 1-4, wherein the one or more medical analysis tasks comprise determining a confidence interval associated with another task of the one or more medical analysis tasks.

7. The computer-implemented method of one of claims 1-6, wherein outputting results of the one or more medical analysis tasks comprises:
presenting a user interface (400) depicting a likelihood ratio score and a cohort of patients similar to the patient.

8. The computer-implemented method of one of claims 1-7, wherein the encoder network and the one or more decoder networks are trained by simulating certain data (508) for one or more biomarkers of the set of biomarkers as being missing in training patient data (512).

9. The computer-implemented method of one of claims 1-7, wherein the encoder network and the one or more decoder networks are trained using deep reinforcement learning by iterating over a window (608) of training patient data (602) and estimated future patient data (606).

10. An apparatus comprising:

means for receiving (202) patient data (108, 302) of a patient for a set of biomarkers (102, 306) acquired at one or more time points within a period of time (112);
means for encoding (204) the patient data using an encoder network (310) to generate patient data embeddings (312);
means for performing (206) one or more medical analysis tasks based on the patient data embeddings using one or more decoder networks (314), the one or more medical analysis tasks comprising generating recommendations for a clinical course of action; and
means for outputting (208) results of the one or more medical analysis tasks.

11. The apparatus of claim 10, wherein the patient data is missing data (110, 304) for certain biomarkers of the set of biomarkers for certain time points within the period of time, and performing one or more medical analysis tasks based on the patient data embeddings using one or more decoder networks comprises:
means for imputing the missing data for the certain biomarkers for the certain time points.

12. The apparatus of claim 10 or 11, wherein the patient data is encoded with time embeddings (104, 308) defining a relative temporal position relative to a given reference time (316), and encoding the patient data using an encoder network to generate patient data embeddings comprises:
means for encoding the patient data encoded with the time embeddings using the encoder network to generate the patient data embeddings.

13. The apparatus of claim 10, 11 or 12, wherein the encoder network comprises a transformer-based encoder network and the one or more decoder networks comprise one or more transformer-based decoder networks.

14. The apparatus of one of claims 10-13, wherein the one or more medical analysis tasks comprise determining a risk score associated with a disease.

15. A non-transitory computer readable medium storing computer program instructions, the computer program instructions when executed by a processor cause the processor to perform operations comprising:

receiving (202) patient data (108, 302) of a patient for a set of biomarkers (102, 306) acquired at one or more time points within a period of time (112);
encoding (204) the patient data using an encoder network (310) to generate patient data embeddings (312);
performing (206) one or more medical analysis tasks based on the patient data embeddings using one or more decoder networks (314), the one or more medical analysis tasks comprising generating recommendations for a clinical course of action; and
outputting (208) results of the one or more medical analysis tasks.

16. The non-transitory computer readable medium of claim 15, wherein the patient data is missing data (110, 304) for certain biomarkers of the set of biomarkers for certain time points within the period of time, and performing one or more medical analysis tasks based on the patient data embeddings using one or more decoder networks comprises:
imputing the missing data for the certain biomarkers for the certain time points.

17. The non-transitory computer readable medium of claim 15 or 16, wherein the one or more medical analysis tasks comprise determining a confidence interval associated with another task of the one or more medical analysis tasks.

18. The non-transitory computer readable medium of one of claims 15-17, wherein outputting results of the one or more medical analysis tasks comprises:
presenting a user interface (400) depicting a likelihood ratio score and a cohort of patients similar to the patient.

19. The non-transitory computer readable medium of one of claims 15-18, wherein the encoder network and the one or more decoder networks are trained by simulating certain data (508) for one or more biomarkers of the set of biomarkers as being missing in training patient data (512).

20. The non-transitory computer readable medium of one of claims 15-18, wherein the encoder network and the one or more decoder networks are trained using deep reinforcement learning by iterating over a window (608) of training patient data (602) and estimated future patient data (606).

EP 4 517 776 A1

# FIG 1

100

Diagnosis code relevant
to the disease

Diagnosis or
Procedure
codes

106

102 {
Biomarker 1
Biomarker 2
Biomarker 3
Biomarker 4
Biomarker 5

Time
Embedding

104

Time

112

Patient data - 108

Missing data - 110

# FIG 2

200

Receive patient data of a patient for a set of biomarkers acquired at one
or more time points within a period of time
202

Encode the patient data using an encoder network to generate patient data embeddings
204

Perform one or more medical analysis tasks based on the patient data embeddings using one
or more decoder networks, the one or more medical analysis tasks comprising generating
recommendations for a clinical course of action
206

Output results of the one or more medical analysis tasks
208

FIG 3

300

308

Time Embedding  Biomarker 1  Biomarker 2  Biomarker 3  Biomarker 4  Biomarker 5

316

306

310 Encoders

312 Embeddings

314-A Risk score decoder for disease 1

314-B Risk score decoder for disease 2

314-C Best next markers to be measured

Patient data - 302

Missing data - 304

# FIG 4

<u>400</u>

Likelihoods

🫁 ⊘ Lung Cancer

low        LR Score = 0.07

Patient has <u>93% decreased</u> chances of
<u>Positive</u> Diagnosis as compared to
<u>Prevalence</u> in screening cohort

Base Cohort (Prevalence)

29        16390
Has Cancer        No Cancer

Similar Patients Cohort

0        354
Has Cancer        No Cancer

🫀 ⊘ Liver Cancer

low        LR Score = 0.65

Patient has <u>35% decreased</u> chances of
<u>Positive</u> Diagnosis as compared to
<u>Prevalence</u> in screening cohort

Base Cohort (Prevalence)

6        17919
Has Cancer        No Cancer

Similar Patients Cohort

0        1809
Has Cancer        No Cancer

⊗ Colorectal Cancer

high        LR Score = 30

Patient has <u>3696% increased</u> chances of
<u>Positive</u> Diagnosis as compared to
<u>Prevalence</u> in screening cohort

Base Cohort (Prevalence)

58        13742
Has Cancer        No Cancer

Similar Patients Cohort

3        16
Has Cancer        No Cancer

EP 4 517 776 A1

# FIG 5

Legend:
- Diagnosis/Procedure Codes (optional) – 502
- Patient data – 504
- Missing data – 506
- Simulated missing data – 508
- Imputed patient data – 510

Rows: Diagnosis or Procedure codes, Biomarker 1, Biomarker 2, Biomarker 3, Biomarker 4, Biomarker 5, Time Embedding

Reference numerals: 500, 512, 514, 516, 508, 510

Transitions: Simulate missing → Impute missing

EP 4 517 776 A1

# FIG 6

600

606

Patient disease risk profile

Next test/procedure recommendation

602

604 — AI algorithm

Diagnosis codes

Procedures codes

Biomarkers (Labs, Demographics)

Time Embedding

608

Outpatient          Inpatient          Outpatient

Patient data

Missing data

# FIG 7

700

702 → Diagnosis codes

Procedures codes

Biomarkers (Labs, Demographics)

Time Embedding

T-n ··· T

704 → Encoders ▷ 706 Embeddings ▷ 708 Decoders

710 → Diagnosis codes

Procedures codes

Biomarkers (Labs, Demographics)

Time Embedding

T-n ··· T+n

# FIG 8

800

826

806

832

808

810

828

834

824

830

802 — $X^{(1)}_1$

804 — $X^{(1)}_2$

$X^{(2)}_1$

$X^{(2)}_2$

$X^{(2)}_3$

$X^{(2)}_4$

$X^{(2)}_5$

$X^{(3)}_1$ — 816

$X^{(3)}_2$ — 818

$X^{(3)}_3$ — 820

$X^{(4)}_1$

822

836

812

814

# FIG 9

900

EP 4 517 776 A1

# FIG 10

1002

Network interface 1006

I/O 1008

Processor 1004

Storage 1012

Memory 1010

Image Acquisition Device 1014

Europäisches
Patentamt

European
Patent Office

Office européen
des brevets

# EUROPEAN SEARCH REPORT

**Application Number**

EP 24 19 7236

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Mowlaei Mohammad Erfan ET AL: "Split-Transformer Impute (STI): Genotype Imputation Using a Transformer Based Model", bioRxiv, 6 March 2023 (2023-03-06), XP093237416, DOI: 10.1101/2023.03.05.531190 Retrieved from the Internet: URL:https://www.biorxiv.org/content/10.1101/2023.03.05.531190v1.full.pdf [retrieved on 2025-01-10] | 1,10,15 | INV. G16H50/20 G16H50/30 |
| Y | * the whole document * | 1-20 | |
| Y | JOSEPH TYLER A ET AL: "Inference of Population Structure from Time-Series Genotype Data", THE AMERICAN JOURNAL OF HUMAN GENETICS, AMERICAN SOCIETY OF HUMAN GENETICS , CHICAGO , IL, US, vol. 105, no. 2, 27 June 2019 (2019-06-27) , pages 317-333, XP085753905, ISSN: 0002-9297, DOI: 10.1016/J.AJHG.2019.06.002 [retrieved on 2019-06-27] * the whole document * | 1-20 | |
| A | WO 2022/223451 A1 (ALCHEMAB THERAPEUTICS LTD [GB]) 27 October 2022 (2022-10-27) * paragraph [0092] * * paragraph [0154] - paragraph [0156] * * paragraph [0199] - paragraph [0200] * | 1-20 | |
| A | WO 2023/107297 A1 (ARTERA INC [US]) 15 June 2023 (2023-06-15) * page 3, line 35 - page 4, line 18 * * page 14, line 10 - line 19 * * page 10, line 27 - page 11, line 26 * * page 20, line 23 * | 1-20 | |

**TECHNICAL FIELDS SEARCHED (IPC)**

G16H
G06N

The present search report has been drawn up for all claims

| Place of search | Date of completion of the search | Examiner |
|---|---|---|
| Munich | 14 January 2025 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 7236

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2025

| Patent document cited in search report | Publication date | Patent family member(s) | | Publication date |
|---|---|---|---|---|
| WO 2022223451 A1 | 27-10-2022 | AU | 2022260043 A1 | 16-11-2023 |
| | | CA | 3215778 A1 | 27-10-2022 |
| | | CN | 117337470 A | 02-01-2024 |
| | | EP | 4327328 A1 | 28-02-2024 |
| | | IL | 307832 A | 01-12-2023 |
| | | JP | 2024514691 A | 02-04-2024 |
| | | KR | 20240011144 A | 25-01-2024 |
| | | US | 2024203523 A1 | 20-06-2024 |
| | | WO | 2022223451 A1 | 27-10-2022 |
| WO 2023107297 A1 | 15-06-2023 | CN | 119278488 A | 07-01-2025 |
| | | EP | 4445389 A1 | 16-10-2024 |
| | | JP | 2024545646 A | 10-12-2024 |
| | | US | 2024395407 A1 | 28-11-2024 |
| | | WO | 2023107297 A1 | 15-06-2023 |

EPO FORM P0459

For more details about this annex : see Official Journal of the European Patent Office, No. 12/82